# EUROPÄISCHE PATENTANMELDUNG

(11) **EP 1 639 968 A1**
(43) Veröffentlichungstag der Anmeldung: **29.03.2006**
(21) Anmeldenummer: 04022670.6
(22) Anmeldetag: 23.09.2004
(51) Int. Cl.: A61F 2/44

(54) **Implantat mit einem in eine Knochenhöhlung einzusetzenden und darin zu verankernden Teil**

(71) Anmelder: Cervitech, Inc., Rockaway, NJ 07866 (US)
(72) Erfinder: Keller, Arnold, 23863 Kayhude (DE)
(74) Vertreter: Glawe, Delfs, Moll

(57) **Zusammenfassung**

Implantat mit einem in eine Knochenhöhlung einzusetzenden Teil, der mindestens einen seitlichen Vorsprung zur Verankerung der angrenzenden Knochensubstanz aufweist. Um einen großen Verankerungsvorsprung (15) verwenden zu können und das Implantat dennoch leicht in die Knochenhöhlung einsetzen zu können, ist der Verankerungsvorsprung (15) zwischen einer zurückgezogenen Implantationsstellung und einer vorragenden Verankerungsstellung bewegbar. Bei dem Implantat kann es sich insbesondere um eine Prothese zum Teilersatz eines Wirbelkörpers handeln.

## Beschreibung

Implantate zum Ersatz eines Teils des Skeletts, beispielsweise eines Gelenks oder eines Knochenabschnitts, müssen häufig mit den angrenzenden Knochenteilen fest verbunden werden. Dies kann dadurch geschehen, daß in dem Knochen eine Höhlung geschaffen wird, die einen Teil des Implantats aufnimmt, das darin verankert wird. Es ist bekannt, die Verankerung durch Knochenzement zu bewirken oder durch Reibhaftung, die dadurch erzeugt wird, daß der Knochen das Implantat mit Spannung umschließt. Dabei können auch kleine Vorsprünge oder Rauhigkeiten an der Implantatoberfläche hilfreich sein. Wenn diese Mittel nicht genügen, muß man zu zusätzlichen Verankerungsmitteln greifen, beispielsweise einer Verschraubung, die aber in vielen Fällen ungeeignet ist. Dies gilt besonders bei Prothesen für den Teilersatz eines Wirbelkörpers (DE-A-4109941, Fig. 2). Diese umfassen eine obere Anschlußplatte zur Verbindung mit einem oberen Wirbelkörper, eine untere Anschlußplatte zur Verbindung mit einem unteren Wirbelkörper und dazwischen einen Überbrückungsteil zum Überbrücken des teilzuersetzenden Wirbelkörpers. Wenn der teilzuersetzende Wirbelkörper lediglich auf der Wirbelbogenseite noch einigermaßen vollständig erhalten ist, wird auf seiner Vorderseite eine Knochenhöhlung geschaffen, in die der Überbrückungsteil eingesetzt wird. Zur festen Verbindung mit dem teilzuersetzenden Wirbelkörper weist er seitlich vorspringende Stege auf, die ein Langloch zur Aufnahme einer Befestigungsschraube enthalten. Die Befestigung der Prothese am teilzuersetzenden Wirbelkörper bestimmt zusätzlich zu den Facettengelenken dessen Stellung zu den benachbarten Wirbelkörpern. Nur wenn seine zur Anlage der Befestigungsstege bestimmten Vorderflächen so bearbeitet sind, daß der Wirbel nach der Verbindung mit den Stegen seine durch die Facettengelenke vorgegebene natürliche Stellung beibehalten kann, besteht Aussicht auf einen beschwerdefreien Sitz der Prothese. Eine solche genaue Bearbeitung ist schwer zu erreichen.

Der Erfindung liegt die Aufgabe zugrunde, ein Implantat mit einer leicht einzusetzenden und wirksamen Verankerungseinrichtung zu schaffen.

Die erfindungsgemäße Lösung besteht in den Merkmalen des Anspruchs 1 sowie vorzugsweise denjenigen der Unteransprüche. Danach ist vorgesehen, daß der in eine Knochenhöhlung einzusetzende Teil des Implantats (dies kann auch die Gesamtheit des Implantats sein) einen seitlichen Verankerungsvorsprung aufweist, der zwischen einer zurückgezogenen Implantationsstellung und einer vorragenden Verankerungsstellung bewegbar ist.

Dabei wird vorausgesetzt, daß der in die Knochenhöhlung einzusetzende Teil eine Form hat, die der Form der Höhlung etwa entspricht, so daß dann, wenn der Verankerungsvorsprung in die vorragende Verankerungsstellung bewegt ist, er hinreichend tief in die umgebende Knochensubstanz hineinragt, um eine Verankerung zu bilden, die die Relativstellung des Implantats zum Knochen sichert. Vorzugsweise hat der in die Knochenhöhlung einzusetzende Teil eine hinterschnittfreie, sich in der Einsetzrichtung verjüngende Form, damit die Knochenhöhlung mit größtmöglicher Übereinstimmung mit der Form dieses Teils hergestellt werden kann. Dabei werden solche Formen bevorzugt, die im Knochen auf möglichst einfache Weise und genau hergestellt werden können. Ein Beispiel dafür ist eine kreiskonische Form, die mit einem Drehwerkzeug hergestellt werden kann, oder eine von ebenen Flächen begrenzte Form, die mittels einer Knochensäge und ggf. einer diese führenden Lehre erzeugt werden kann.

Die Form der Knochenhöhlung und die entsprechende Form des einzusetzenden Teils bestimmen diejenige Richtung, in welcher der darin eingesetzte Teil entweichen kann, wenn er nicht verankert ist. Dies ist im allgemeinen auch die Einsetzrichtung. Da der Vorsprung die Bewegung in dieser Richtung verhindern soll, muß er quer zu dieser Richtung in die Knochensubstanz eingreifen. Dies setzt im allgemeinen voraus, daß er seitlich in bezug auf diese Richtung am Implantat angeordnet ist.

Da der Verankerungsvorsprung bei der Implantation in den einzusetzenden Teil zurückgezogen ist, hindert er die Implantation nicht. Erst danach wird er in die vorragende Verankerungsstellung bewegt.

Vorzugsweise sind mehrere Verankerungsvorsprünge vorgesehen, die nach einem weiteren Merkmal der Erfindung durch denselben Mechanismus bewegbar sind. Dies erleichtert die Operation, weil nur ein einziger Bewegungsschritt erforderlich ist, um alle gemeinsam bewegbaren Verankerungsvorsprünge in die Verankerungsstellung zu überführen. Dafür kann ein Treibglied vorgesehen sein, das auf alle zugehörigen Verankerungsvorsprünge wirkt. Besonders vorteilhaft ist es, wenn es die Verankerungsvorsprünge in unterschiedlicher Richtung verschiebt, beispielsweise zwei Verankerungsvorsprünge in entgegengesetzter Richtung oder vier Verankerungsvorsprünge in um 90° zueinander versetzten Richtungen.

Das Treibglied ist vorzugsweise so ausgebildet, daß es durch Drehung die Bewegung der Verankerungsvorsprünge bewirken kann. Es kann z.B. als Exzenter ausgebildet sein oder als Drehnocken, der in derjenigen Drehstellung, die der Implantationsstellung der Vorsprünge entspricht, weniger weit zu den Vorsprüngen hin vorragt als in derjenigen Drehstellung, die der Verankerungsstellung entspricht. Eine besonders vorteilhafte Ausführungsform des Treibglieds ist ein Zahnritzel, das in Zahnstangen eingreift, die mit den Verankerungsvorsprüngen verbunden sind. Dies hat den Vorteil, daß die Schubstrecke der Vorsprünge zwischen der Implantationsstellung und der Verankerungsstellung nicht auf die Nockenhöhe des Treibglieds begrenzt ist.

Die Verankerungsvorsprünge bzw. das zu deren Bewegung verwendete Treibglied sind zweckmäßigerweise von derjenigen Seite des Implantats her betätigbar, von der das Implantat in die Knochenhöhlung eingesetzt wird.

Damit die Verankerung des Implantats im Knochen nicht nur durch die Verankerungsvorsprünge stattfindet, ist der in die Knochenhöhlung einzusetzende Teil zweckmäßigerweise so ausgebildet, daß sich das Knochengewebe innig mit seiner Oberfläche verbinden kann. Vorzugsweise weist er auch Öffnungen oder Poren für die Aufnahme von Knochengewebe auf.

Es kann ein Hohlraum vorgesehen sein, der durch die Öffnungen bzw. Poren zugänglich ist und einer Füllung von Knochenmaterial (einschließlich Knochenersatzmaterial) versehen ist oder versehen werden kann.

Wenn es sich um den Fall des Teilersatzes eines Wirbelkörpers handelt, wird in diesem von der Vorderseite her eine Knochenhöhlung geschaffen, die zu der Gestalt des Überbrückungsteils paßt und diesen im wesentlichen vollständig aufnimmt. Damit dies möglich ist, wird der Überbrückungsteil schmaler als der Wirbelkörper ausgebildet. Durch den gegenseitigen Formschluß stützen sich der Überbrückungsteil und der Wirbelkörper gegenseitig. Der Überbrückungsteil enthält die vorerwähnten seitlichen Verankerungsvorsprünge, die ihn in der Knochenhöhlung festhalten.

Während sich die Vorsprünge in ihrer zurückgezogenen Implantationsstellung befinden, läßt sich das Implantat leicht in die Knochenhöhlung einsetzen. Danach werden sie herausgeschoben, dringen in das beiderseits des Implantats befindliche Knochengewebe ein und erreichen dadurch ihre Verankerungsstellung. Zweckmäßigerweise sind sie von der Vorderseite des Überbrückungsteils her betätigbar.

Besondere Vorteile hat die Erfindung in der Anwendung bei der Halswirbelsäule.

Die Erfindung wird im folgenden näher unter Bezugnahme auf die in der Zeichnung dargestellten Ausführungsbeispiele erläutert. Es zeigen:
- Fig. 1: einen Längsschnitt eines ersten Ausführungsbeispiels in der Medianebene,
- Fig. 2: eine Frontalansicht desselben Implantats,
- Fig. 3: die perspektivische Ansicht eines zweiten Ausführungsbeispiels von schräg vorne,
- Fig. 4: eine Schnittdarstellung des Ausführungsbeipiels von Fig. 3,
- Fig. 5: einen Schnitt durch ein drittes Ausführungsbeispiel,
- Fig. 6: den Schnitt gemäß Fig. 5 in einer anderen Funktionsstellung,
- Fig. 7: einen Schnitt gemäß Schnittlinie V der Fig. 6,
- Fig. 8: eine Vorderansicht eines vierten Ausführungsbeispiels,
- Fig. 9: einen Frontalschnitt,
- Fig. 10: eine Draufsicht,
- Fig. 11: eine konstruktive Einzelheit und
- Fig. 12: das äußere Ende eines Verankerungsvorsprungs.

Die Ausführungsbeispiele betreffen eine Prothese zum Teilersatz eines Wirbelkörpers. Es versteht sich aber, daß die Erfindung auch bei anderen Prothesen anwendbar ist, beispiesweise Zahnimplantaten.

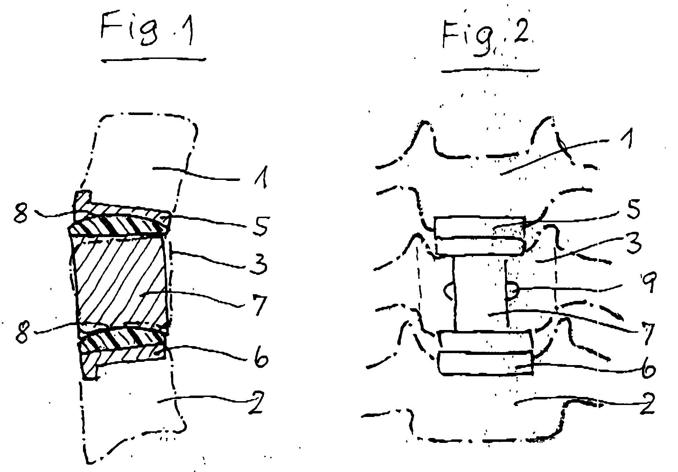

Fig. 2 zeigt einen oberen Wirbelkörper 1 und einen unteren Wirbelkörper 2, zwischen denen sich ein teilzuersetzender Wirbelkörper 3 befindet. Zwischen dem oberen und dem unteren Wirbelkörper ist eine Prothese eingesetzt. Sie umfaßt eine obere Anschlußplatte 5, die mit dem oberen Wirbelkörper 1 verbunden ist, eine untere Anschlußplatte 6, die mit dem unteren Wirbelkörper 2 verbunden ist, und einen Überbrückungsteil 7, der die Platten 5 und 6 verbindet. Zwischen den Anschlußplatten 5 und 6 und dem Überbrückungsteil 7 befindet sich jeweils ein Gelenk (beispielsweise gemäß EP-A-560140) mit einer Gelenkfläche 8. Diese wird im oberen Gelenk einerseits von der Unterseite der Anschlußplatte 5 und andererseits von einem Gelenkteil 4 gebildet, der mit dem Überbrückungsteil 7 in nicht dargestellter Weise verbunden ist. Im unteren Gelenk wird sie einerseits von der Unterseite des Überbrückungsteils 7 und andererseits von dem Gelenkteil 4 gebildet, der mit der unteren Anschlußplatte 6 in nicht dargestellter Weise verbunden ist. Statt eines Gelenks mit sphärischer Gelenkfläche kann auch ein anderer Gelenktyp verwendet werden, beispielsweise ein solcher mit flexiblem Kissen (DE-U-20115281) oder mit Biegefeder (DE-A-4109941). Falls der obere Wirbelkörper 1 und der untere Wirbelkörper 2 starr verbunden werden sollen, können die Gelenke auch gänzlich entfallen. Schließlich ist es möglich, nur ein Gelenk zwischen der oberen Anschlußplatte 5 und dem Überbrückungsteil 7 oder zwischen der unteren Anschlußplatte 6 und dem Überbrückungsteil 7 zu verwenden.

Während die Anschlußplatten 5 und 6 eine übliche Größe haben, die im Interesse geringer Druckkräfte zwischen den Anschlußplatten und den zugehörigen Wirbelkörpern bemessen ist, hat der Überbrückungsteil 7 eine Breite, die beträchtlich geringer ist als diejenige des teilzuersetzenden Wirbelkörpers 3. Dies schafft die Möglichkeit, den Überbrückungsteil in eine von der Vorderseite in den Wirbelkörper 3 eingearbeitete Knochenhöhlung einzusetzen. Der Überbrückungsteil bildet im Sinne der Ansprüche den in die Knochenhöhlung einzusetzenden Teil. Die Knochenhöhlung hat eine Gestalt, die möglichst genau mit der Gestalt des Überbrückungsteils 7 übereinstimmt. Dadurch wird eine möglichst vollflächige, spielfreie Anlage der Implantatoberfläche an der künstlich geschaffenen Oberfläche des Knochens möglich. Zum einen ergibt dies eine im wesentlichen spielfreie gegenseitige Abstützung. Zum anderen ergibt sich daraus die Möglichkeit einer durch Knochenwachstum entstehenden stabilen Verbindung zwischen dem Knochen und dem Implantat. Und schließlich gibt dies Sicherheit dafür, daß die am Implantat vorgesehenen Verankerungsvorsprünge 9 mit im wesentlichen ihrer vollen Länge in das Knochengewebe eingreifen, um die Verankerungskräfte übertragen zu können.

Der Begriff Knochenhöhlung soll keinerlei Einschränkung bezüglich der Form beinhalten. Insbesondere gilt dies für die Tiefe und die seitliche Geschlossenheit der Knochenhöhlung. Sie braucht nur so tief zu sein, daß seitlich neben dem darin eingesetzten Teil so viel Knochenmaterial vorhanden ist, daß daran die Verankerung mittels der Verankerungsvorsprünge stattfinden kann. Sie braucht auch nicht an allen Seiten geschlossen zu sein. In dem hier erläuterten Beispiel ist sie oben und unten offen. Zusätzlich zu den Verankerungsvorsprüngen können Öffnungen 12 oder Poren vorgesehen sein, in denen hineinwachsende Knochensubstanz sich verankern kann. Um diesen Prozeß zu beschleunigen, können die Öffnungen vorab mit Knochenspänen gefüllt werden. Auch eine Beschichtung des Implantats mit osteokonduktiver oder osteoinduktiver Substanz ist möglich.

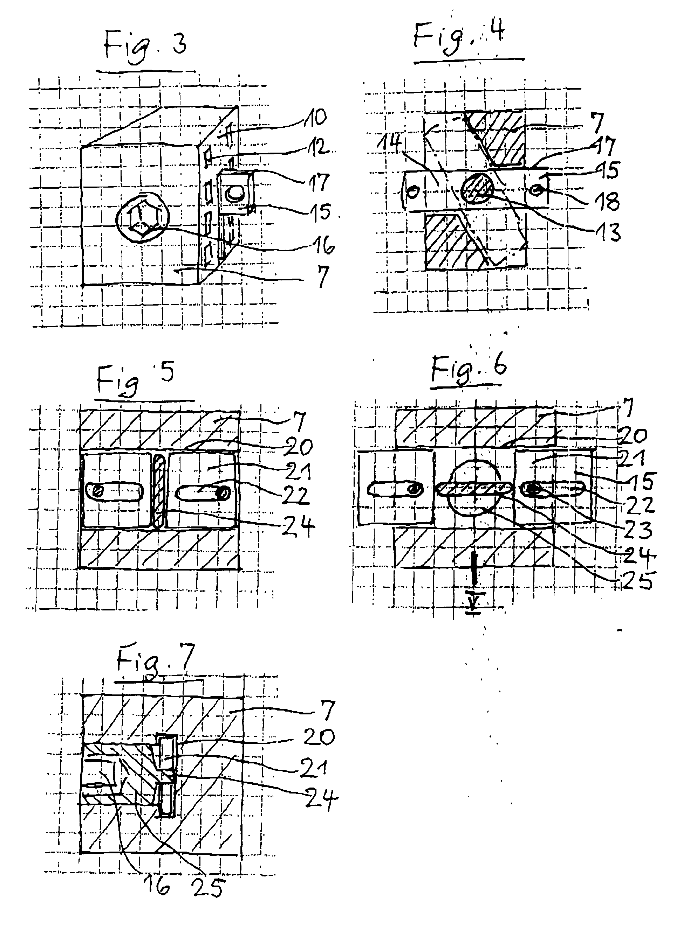

Fig. 3 und 4 zeigen ein Ausführungsbeispiel mit Vorsprüngen 15, die während der Implantation in den Überbrückungsteil zurückgezogen sind und anschließend in die Verankerungsstellung bewegt werden. Der Überbrückungsteil 7 hat einen Querschlitz, der in Fig. 4 als nicht schraffierter Bereich erscheint. Darin ist auf einer Welle 13 ein Riegel 14 gelagert, der in einer ersten, strichpunktiert dargestellten Drehstellung gänzlich in das Implantat zurückgezogen ist und in der mit durchgezogenen Linien dargestellten Drehstellung mit seinen die Vorsprünge 15 bildenden Enden über dessen Seitenflächen 10 hinausragt. In diese Verankerungsstellung wird der mit der Welle 13 drehfest verbundene Riegel 14 mit Hilfe von Schlüsselansatzflächen 16 am vorderen Ende der Welle 13 erst dann gedreht, wenn der Überbrückungsteil die vorgesehene Implantationsstellung erreicht hat. Die Enden 15 des Riegels 14 schneiden während dieser Bewegung in die Knochensubstanz des teilzuersetzenden Wirbels ein und können zur Erleichterung dieses Vorgangs an der vorausgehenden Kante 17 selbstschneidend ausgebildet sein. Im dargestellten Beispiel enthalten die Riegelenden 15 eine Öffnung 18, in welche Knochensubstanz einwächst und dadurch den Riegel 14 an einer Rückbewegung hindert.

Ein wesentlicher Vorteil der nach dem Einsetzen des Überbrückungsteils ausfahrbaren Vorsprünge besteht darin, daß sie die nachträgliche Korrektur der Position des Überbrückungsteils gestatten. Wenn die Bearbeitung der Knochenhöhlung nicht so genau geschehen sein sollte, wie es wünschenswert wäre, geben sie die Möglichkeit, den Überbrückungsteil mit Spiel einzusetzen und die genaue Positionierung den Vorsprüngen zu überlassen, die dann ausgefahren werden, wenn er seine anatomisch richtige Stellung erreicht hat.

Das Implantat kann mehrere parallel mit Abstand hintereinander angeordnete Schlitze mit darin auf der Welle 13 angeordneten Riegeln 14 enthalten, die durch die Bewegung der Welle 13 gemeinsam in den Verriegelungszustand überführt werden und dann in einem geeigneten Axialabstand hintereinander in die Knochensubstanz eingreifen.

Ein weiteres Ausführungsbeispiel von beweglichen Verankerungsvorsprüngen zeigen Fig. 5 bis 7. In dem Überbrückungsteil 7 ist ein quer verlaufender Schlitz 20 enthalten, der als Führung für zwei Riegel 21 dient, die darin die in Fig. 5 und 6 dargestellten Endstellungen einnehmen können. Die Enden der Riegel bilden die Verankerungsvorsprünge 15. Sie enthalten ein Langloch 22, das in der Verschieberichtung verläuft und von einem im Überbrückungsteil festen Stift 23 durchdrungen ist. Er verhindert, daß sie weiter aus dem Implantat herauswandern können, als es der in Fig. 6 dargestellten Verankerungsstellung entspricht.

Für die Überführung der Riegel 21 aus der Implantationsstellung gemäß Fig. 5 in die Verankerungsstellung gemäß Fig. 6 dient ein Spreizglied, das in diesem Ausführungsbeispiel als Drehnocken 24 ausgebildet ist, der am Ende einer Welle 25 fest angeordnet ist. Wird er aus der Stellung der Fig. 5, in welcher er schmal zwischen den Riegeln 21 liegt, um 90 ° in die Stellung der Fig. 6 gedreht, so spreizt er die Riegel 21 auseinander, bis ihre Enden als Verankerungsvorsprünge über das Implantat vorragen und es im Knochen verankern. Die Welle 25 ist an ihrem Ende, das in der Vorderfläche des Überbrückungsteils zugänglich ist, mit Schlüsselansatzflächen 16 für ein Drehinstrument versehen. Es können nicht dargestellte Mittel vorgesehen werden, die die Welle 25 im Überbrückungsteil 7 festhalten und sie in der Verankerungsstellung gemäß Fig. 6 sichern.

Fig. 10 zeigt den Aufbau des Überbrückungsteils 7 des vierten Ausführungsbeispiels. Er wird von einem schmalen Kernteil 29 und einem Käfig 30 gebildet, der den Kernteil 29 mit Abstand umgibt. An der Oberseite des Kernteils 29 ist der Gelenkteil 4 (siehe Fig. 1) befestigt, der mit einer nicht gezeigten Anschlußplatte 5 ein oberes Gelenk bildet. An seiner Unterseite ist eine Gelenkfläche 8 vorgesehen, die mit einem nicht gezeigten Gelenkteil 4 und einer unteren Anschlußplatte 6 ein nicht gezeigtes unteres Gelenk bildet. Der Käfig enthält eine Vielzahl von Löchern 31, die das Einwachsen von Knochensubstanz ermöglichen. Dies kann gewünschtenfalls dadurch gefördert werden, daß der Zwischenraum zwischen dem Kernteil 29 und dem Käfig 30 mit neutraler oder homologer Knochensubstanz oder einer Knochenersatzsubstanz gefüllt wird. Diese Füllung kann fabrikmäßig vorbereitet sein.

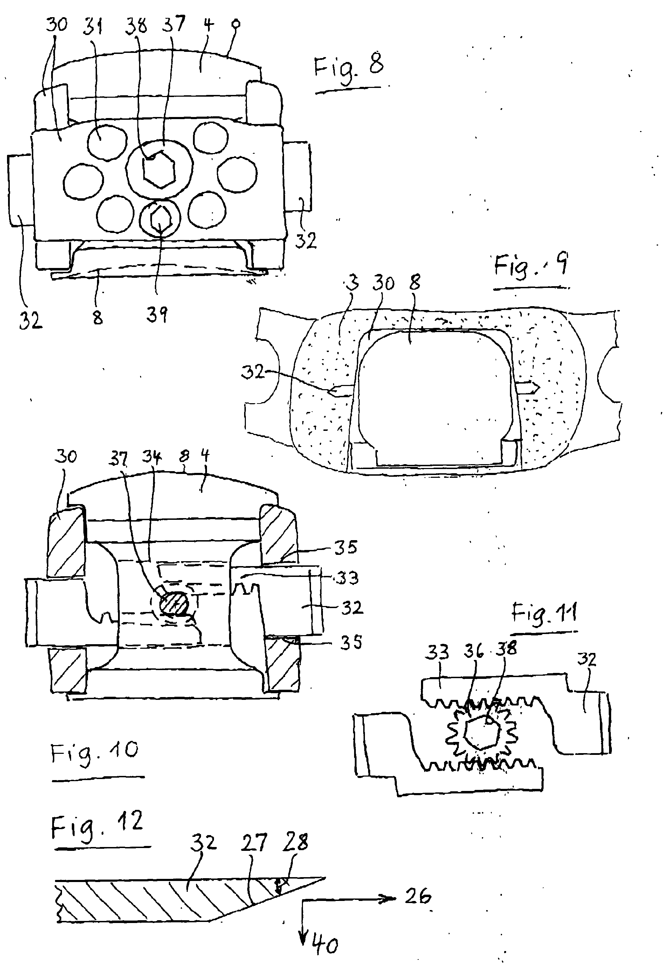

Wie in Fig. 9 erkennbar, ist der Käfig 30 schmaler als der teilzuersetzender Wirbelkörper 3 ausgebildet, so daß er in eine darin gebildete, nach vorne offene Knochenhöhlung eingesetzt werden kann. Seine Querschnittsform wird von ebenen Flächen begrenzt. Dies erleichtert die Herstellung einer zur Form des Käfigs passenden Knochenhöhlung und das genaue Einsetzen des Käfigs.

Aus den Seitenflächen des Überbrückungsteils 7 ragen Vorsprünge 32 hervor, die am Ende angeschärft sind. Außer der in Fig. 8 und 9 dargestellten Verankerungsstellung können sie eine nicht dargestellte Implantationsstellung einnehmen, in der sie in den Überbrückungsteil 7 zurückgezogen sind. Ihre Bewegung von der einen in die andere Stellung wird mit Hilfe des in Fig. 11 dargestellten Mechanismus bewirkt. Dieser enthält als Spreizglied eine Ritzel 36. Dessen Zähne greifen in die Zähne von Zahnstangen 33 ein, die beiderseits des Ritzels angeordnet sind und mit den Verankerungsvorsprüngen 32 fest verbunden sind. Die Zahnstangen 33 und/oder die Vorsprünge 32 sind in Führungsflächen 34 des Kernteils 29 und/oder Führungsflächen 35 des Käfigs 30 in ihrer Längsrichtung verschiebbar gelagert. Das Ritzel 36 oder die Ritzelwelle 37 ist mit Betätigungsflächen 38 versehen, die von der Vorderseite des Implantats für ein Drehinstrument zugänglich sind. Wird das Ritzel im Uhrzeigersinn(gesehen in Fig. 11) gedreht, werden die Zahnstangen 33 mit den Vorsprüngen 32 aus der Montagestellung nach außen in die Verankerungsstellung geschoben. Dies wird dann durchgeführt, wenn das Implantat eingesetzt ist und seine vorgesehene Stellung erreicht hat. Die Vorsprünge dringen dabei in die seitlich der Knochenhöhlung befindlichen Knochenbereiche ein und verankern den Überbrückungsteil 7 dadurch darin.

Damit sich die Vorsprünge nicht während des Gebrauchs der Prothese ungewollt aus der Verankerungsstellung zurückziehen, ist eine Sicherungseinrichtung vorgesehen. Diese ist als Sicherungsschraube 38 ausgebildet, die in dem Kernteil 29 in eine die Bewegung der Zahnstangen verhindernde Sicherungsstellung geschraubt werden kann, sobald die Vorsprünge die Verankerungsstellung erreicht haben. Sie kann beispielsweise gegen eine Zahnstange oder gegen das Ritzel pressen, um diese durch Reibung an Bewegung zu hindern, oder sie kann in den Zwischenraum zwischen dem Ritzel 36 und einem Vorsprung 32 oder zwischen die kämmenden Zähne von Ritzel 36 und Zahnstange 33 eingreifen, um sie auf diese Weise formschlüssig in der Verankerungsstellung zu verriegeln.

Gemäß Fig. 12 ist das äußere Ende eines Verankerungsvorsprungs 15 oder 32 mit einer Fläche 27 versehen, die geneigt gegenüber der Richtung 26 verläuft, in der der Verankerungsvorsprung aus dem Implantat in die Verankerungsstellung ausgeschoben wird. Sie wird deshalb als Schrägfläche bezeichnet. Während des Auschiebens übt die umgebende Knochensubstanz eine Kraftkomponente quer zur Ausschubrichtung 26 im Sinne des Pfeils 40 auf diese Schrägfläche aus. Diese Kraftkomponente wird von dem Implantat aufgenommen, wodurch das Implantat in der Richtung dieser Kraft gedrückt wird. Dies kann dazu genutzt werden, den von der Knochenhöhlung aufzunehmenden Teil weiter in die Knochenhöhlung hineinzudrängen, um einen möglichst festen Sitz zu erreichen.

Voraussetzung für diese Wirkung der Schrägfläche ist, daß nicht an der gegenüberliegenden Seite des Verankerungsvorsprungs eine gegensinnig gleiche Schrägfläche vorhanden ist. Im Beispiel der Fig. 9, in dem der Verankerungsvorsprung zwei gegensinnig gleiche Schneidenfacetten bildet, heben sich die von diesen Facetten erzeugten Kräfte gegenseitig auf. Im Gegensatz dazu zeichnet sich die erfindungsgemäße Schrägfläche also durch das Merkmal aus, daß sie einseitig angeordnet ist. Dies trifft nicht nur auf Fälle zu, in denen auf der gegenüberliegenden Seite überhaupt keine Schrägfläche vorhanden ist, sondern auch auf Fälle, in denen eine gegenüberliegende Schrägfläche eine geringere Kraftkomponente erzeugt, weil sie beispielsweise kleiner oder ihr Winkel ungünstiger ist.

Im Beispiel gemäß Fig. 12 bildet die Schrägfläche 27 eine Facette einer Schneide. Dies ist aber nicht Voraussetzung für ihre Wirkung. Beispielsweise könnte ein blattförmiger Verankerungsvorsprung statt dessen in seiner Gesamtheit schräg zurRichtung seiner Blattfläche geführt werden.

## Patentansprüche

1. Implantat mit einem in eine Knochenhöhlung einzusetzenden Teil, der mindestens einen seitlichen Vorsprung zur Verankerung in der angrenzenden Knochensubstanz aufweist, **dadurch gekennzeichnet, daß** der Verankerungsvorsprung (9, 15, 32) zwischen einer zurückgezogenen Implantationsstellung und einer vorragenden Verankerungsstellung bewegbar ist.

2. Implantat nach Anspruch 1, **dadurch gekennzeichnet, daß** der mindestens eine Verankerungsvorsprung (9, 15, 32) eine einseitige Schrägfläche (27) aufweist, die einen Winkel (28) mit der Ausschubrichtung (26) des Verankerungsvorsprungs einschließt.

3. Implantat nach Anspruch 2, **dadurch gekennzeichnet, daß** der Winkel (28) so angeordnet ist, daß das Implantat beim Ausschieben des Verankerungsvorsprungs in die Knochenhöhlung gedrückt wird.

4. Implantat nach Anspruch 1, **dadurch gekennzeichnet, daß** mehrere durch denselben Mechanismus bewegbare Verankerungsvorsprünge vorgesehen sind.

5. Implantat nach Anspruch 4, **dadurch gekennzeichnet, daß** zur Verschiebung der Verankerungsvorsprünge (15, 32)ein Treibglied (24, 36) vorgesehen ist, das bei seiner Betätigung die Verankerungsvorsprünge (15, 32) in unterschiedlichen Richtungen verschiebt.

6. Implantat nach Anspruch 5, **dadurch gekennzeichnet, daß** das Treibglied als Drehnocken (24) ausgebildet ist, der zwischen mindestens zwei Verankerungsvorsprüngen (15) oder damit fest verbundenen Teilen (21) angeordnet ist und diese bei Drehung spreizt.

7. Implantat nach Anspruch 5, **dadurch gekennzeichnet, daß** das Treibglied als Ritzel (36) ausgebildet ist, das in mit den Verankerungsvorsprüngen (32) verbundene Zahnstangen (33) eingreift.

8. Implantat nach einem der Ansprüche 1 bis 7, **dadurch gekennzeichnet, daß** die Verankerungsvorsprünge (15, 32) von derjenigen Seite des Implantats her betätigbar sind, von der her das Implantat in die Knochenhöhlung einzusetzen ist.

9. Implantat nach Anspruch 1, **dadurch gekennzeichnet, daß** der in die Knochenhöhlung einzusetzende Teil (7) Öffnungen (12) oder Poren für die Aufnahme von Knochengewebe aufweist.

10. Implantat nach Anspruch 9, **dadurch gekennzeichnet, daß** der in die Knochenhöhlung einzusetzende Teil mit einer Füllung von Knochenmaterial versehen ist.

11. Implantat nach einem der Ansprüche 1 bis 10, **dadurch gekennzeichnet, daß** es eine Prothese zum Teilersatz eines Wirbelkörpers ist mit einer oberen Anschlußplatte (5) zur Verbindung mit einem oberen Wirbelkörper (1), einer unteren Anschlußplatte (6) zur Verbindung mit einem unteren Wirbelkörper (2) und einem die obere und die untere Anschlußplatte (5, 6) verbindenden Überbrückungsteil (7), der zum Überbrücken mindestens eines zwischen dem oberen und dem unteren Wirbelkörper (1, 2) befindlichen teilzuersetzenden Wirbelkörpers (3) und zur Befestigung an diesem ausgebildet ist, **dadurch gekennzeichnet, daß** der Überbrückungsteil (7) zur Befestigung in einer Knochenhöhlung des teilzuersetzenden Wirbelkörpers (3) seitlich vorragende Verankerungsvorsprünge (9, 15, 32) aufweist, die zwischen einer zurückgezogenen Implantationsstellung und einer vorragenden Verankerungsstellung bewegbar sind.

12. Implantat nach Anspruch 11, **dadurch gekennzeichnet, daß** die Verankerungsvorsprünge (15, 32) von der Vorderseite des Überbrückungsteils (7) her betätigbar sind.
